# EUROPEAN PATENT APPLICATION

(11) **EP 1 580 276 A1**
(43) Date of publication of application: **28.09.2005**
(21) Application number: 03812371.7
(22) Date of filing: 02.12.2003
(51) Int. Cl.: C12N 15/869, C12N 5/10

(54) **PACKAGING CELL SYSTEM FOR EB VIRUS VECTOR**

(30) Priority: 02.12.2002 JP 2002349467
(71) Applicant: Evec Incorporated, Hokkaido 060-0042 (JP)
(72) Inventor: TAKADA, Kenzo, Sapporo-shi, Hokkaido 001-0904 (JP); YOSHIYAMA, Hironori, Sapporo-shi, Hokkaido 063-0843 (JP)
(74) Representative: Vossius & Partner
(86) International application number: PCT/JP2003/015419
(87) International publication number: WO 2004/050886

(57) **Abstract**

A packaging cell is constructed by preparing a packaging cell carrying an EB virus gene lacking a packaging signal but not carrying a wild type EB virus gene having the packaging signal, and introducing an amplicon plasmid having the packaging signal but having no viral replication ability into the cell. By inducing lytic infection of the packaging cell introduced with an amplicon plasmid, an EB virus vector without viral replication ability is produced.

## Description

### TECHNICAL FIELD

The present invention relates to a method for producing an EB virus vector, and a packaging cell system used for the method.

### BACKGROUND ART

In transferring genes into cells, a substance which plays a role in transferring genes into a target cell is called "vector". From the viewpoint of safety, a virus-derived vector from which genes derived from the original wild type virus have better to be removed as much as possible. Epstein-Barr virus (hereinafter, referred to EB virus), which is one of the herpes viruses, has an ability to infect human B lymphocytes and promotes their proliferation. When compared with currently used vectors based on other viruses, the EB virus vector has many advantages of, for example, 1) being to be maintained stably in a transfected cell for a long period of time; 2) ability to incorporate large foreign genes of over 100 kb in size; 3) ability to introduce a gene efficiently into resting B lymphocytes, particularly, primary B lymphocytes; 4) ability to introduce and express a gene into B lymphocytes which are known to work as antigen presenting cells; 5) ability to propagate the B lymphocytes, into which a gene is introduced, *in vitro;* 6) ability to facilitate to prepare a vector efficiently, when a system of preparing a recombinant virus in *E. coli* is used; and the like.

As cell strains producing EB virus, there are known Akata cell, B95-8 cell, P3HR-1 cell and the like. Among them, Akata cell has about 20 copies of circular EB virus genomes in the nucleus, and can induce infectious EB virus production by adding an anti-human immunoglobulin antibody into a liquid culture medium. Therefore, Akata cell is a cell strain suitable to produce EB virus in large amount (Takada K, *Int J Cancer* 33, 27-32 (1984); Takada K and Ono Y, J *Virol* **63,** 445-449 (1989)). A clone in which an EB virus genome was dropped off was obtained by subculture of Akata cell for a long period (hereinafter, referred to as "EB virus-negative Akata cell"). The EB virus-negative Akata cell can be re-infected with EB virus and repeatedly express an ability to produce the virus by treatment with an anti-human immunoglobulin antibody (Japanese Patent Laid-Open No. Hei 7-115966). In addition, an Akata cell having only a recombinant EB virus genome can be produced, by generating a homologous recombinant EB virus having a drug marker gene in Akata cell, inducing EB virus production, and re-infecting an EB virus-negative Akata cell with the resulting virus and then selecting a cell with the drug. This cell has an ability to produce a recombinant EB virus in large amount by treatment with an anti-human immunoglobulin antibody (Japanese Patent Laid-Open No. Hei. 8-009971).

There is a report which mentions methods for integrating a circular EB virus genome originated from EB virus B95-8 strain in an *E. coli* artificial chromosome and for multiplying the integrated form in *E. coli* (US Patent No. 6,291,246). By using this system, mutation can be introduced efficiently even in a gene essential for replication and proliferation of EB virus by homologous recombination in *E. coli.* A research group in Germany discloses a method to introduce an EB virus genome, from which a packaging signal was eliminated in *E. coli* system, into a 293 cell originated from fetal kidney and to apply the cell as a packaging cell for producing EB virus vector (Delecluse HJ, Pich D, Hilsendegen T, Baum C, Hammerschmidt W. *Proc Natl Acad Sci USA* **96**, 5188-5193 (1999)). In the method, EB virus vector production is induced by extraneously and simultaneously introducing an EB virus early gene BZLF1 and amplicon plasmid into the 293 cell and allowing to express them. However, in this system, means for EB virus vector production are complicated, and additionally, the titer of the produced EB virus vector is very low. Therefore, this system is not suitable for production of EB virus vectors in large quantity.

A system for efficient introduction of a foreign gene into a cell by using an EB virus envelope is reported (Delecluse H. J, Hammerschmidt W, *J Clin Pathol: Mol Pathol* 2000; **53**: 270-279, and the like). A method for packaging a gene vector DNA without contamination of any helper virus is also reported (Japanese Patent Laid-Open No. Hei 11-221073).

An object of the present invention is to provide a system for efficient production of a large amount of EB virus vector lacking viral replication ability.

### DISCLOSURE OF THE INVENTION

A method for producing a packaging cell in order to construct a system for producing an EB virus vector lacking viral replication ability is provided.

In the first embodiment, the method of the present invention comprises the steps of:
introducing a gene fragment for homologous recombination lacking a packaging signal into an EB virus-positive Akata cell, thereby deleting a packaging signal of EB virus by homologous recombination, and
cloning a packaging cell carrying an EB virus genome lacking a packaging signal, but not carrying a wild type EB virus genome having a packaging signal.

In the second embodiment, the method of the present invention comprises the steps of:
preparing an EB virus genome lacking a packaging signal in the bacterial E. *coli* cell, introducing the EB virus genome into an EB virus-positive Akata cell, and
cloning a packaging cell carrying an EB virus genome lacking a packaging signal, but not carrying a wild type EB virus genome having a packaging signal.

In the third embodiment, the method of the present invention comprises the steps of:
preparing an EB virus genome lacking a packaging signal by the use of *E. coli,* introducing the EB virus genome into an EB virus-negative Akata cell expressing EBNA1, and
cloning a packaging cell carrying an EB virus genome lacking a packaging signal, but not carrying a wild type EB virus genome having a packaging signal.

In another aspect, the present invention provides a method for constructing an Akata packaging cell introduced with an amplicon plasmid, which is used for producing an EB virus vector lacking viral replication ability. The method comprises the step for introducing an amplicon plasmid having a packaging signal, but lacking viral replication ability, into a packaging cell obtained by any one of the above methods.

In still another aspect, the present invention provides a method for producing an EB virus vector lacking viral replication ability, comprising induction of lytic infection of an Akata packaging cell introduced with an amplicon plasmid, which is produced by the above method of the present invention, thereby allowing an EB virus vector covered with a virus envelope to be released.

In further still another aspect, the present invention provides a method for producing an immortalized B lymphocyte, comprising inducing lytic infection of an Akata packaging cell introduced with an amplicon plasmid, which is produced by the above-mentioned method of the present invention, thereby allowing an EB virus vector covered with a virus envelope to be released, and infecting a B lymphocyte with the resulting EB virus vector.

According to the present invention, it is possible to produce simply and efficiently an EB virus vector lacking viral replication ability through induction of lytic infection of a packaging cell introduced with an amplicon plasmid, which is prepared by introducing an amplicon plasmid into the packaging cell.

### BRIEF DESCRIPTION OF THE DRAWINGS

Figure 1 shows the method for preparing a packaging cell of the present invention.
Figure 2 shows production of an EB virus vector by the Akata packaging cell introduced with an amplicon plasmid.
Figure 3 shows construction of a plasmid for cloning of a packaging signal gene (TR) and a targeting plasmid for disrupting the packaging signal.
Figure 4 shows a position of primers used for confirmation of homologous recombination of the gene.

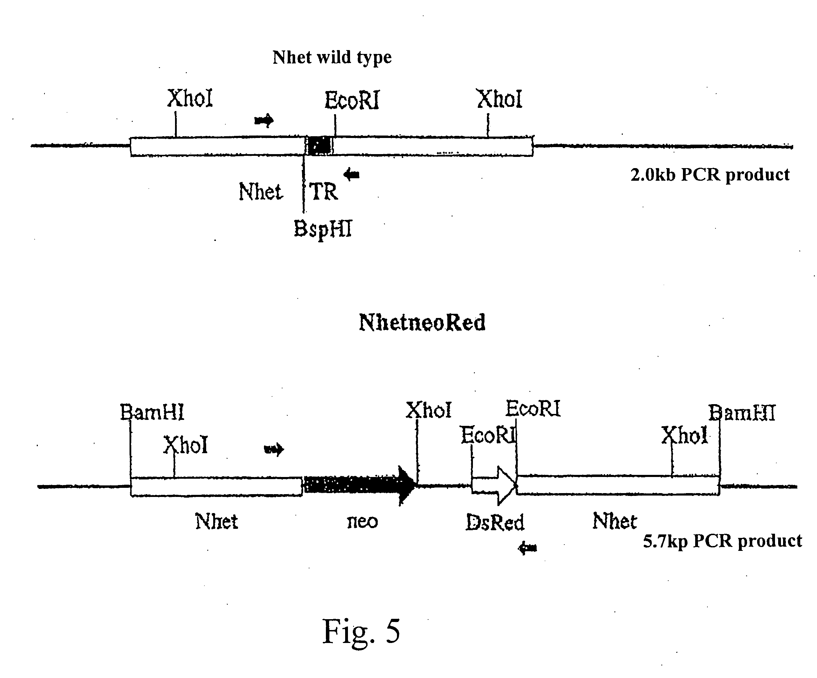

Figure 5 shows a position of primers used for gene amplification in order to confirm dropping off of wild type EB virus.
Figure 6 shows one of the examples for a construction of both TR deficient EB virus genome and amplicon plasmid which is present in the packaging cell introduced with an amplicon plasmid.
Figure 7 shows production of EB virus vector by the packaging cell introduced with an amplicon plasmid.
Figure 8 shows generation of a virus incapable of replicating its own viral genome, and production of the EB virus vector incapable of replicating its own viral genome using a packaging cell into which the virus incapable of replicating its own viral genome is introduced by infection.
Figure 9 shows construction of a targeting plasmid for disruption and deletion of BALF2 gene and a probe used for detecting viruses resulting from homologous recombination.
Figure 10 shows positions of primers used for gene amplification in order to confirm production of a virus lacking BALF2 gene by homologous recombination.

### DETAILED DESCRIPTION OF THE INVENTION

The present invention provides a method for producing a packaging cell used for preparing a system for producing an EB virus vector without viral replication ability.

"Packaging cell" is a cell having an EB virus genome which lacks its packaging signal but is able to express viral proteins necessary for production of virus particles. The packaging signal is a *cis*-acting sequence necessary for each viral genome to be packaged in an EB virus particle, and it is also referred as TR (Terminal Repeat). In the present specification, the term "lacking (or deletion)" includes both a case where the packaging signal is totally deleted and a case where the packaging signal is partially deleted or mutated whereby a function as a packaging signal is lost.

"EB virus vector" means a virus particle covered with an EB virus envelope, having an ability to infect human B lymphocytes. The EB virus vector may contain a foreign gene. "EB virus vector without viral replication ability" means a vector which is able to infect a host cell and replicate the genome in the cell, but unable to be released as an enveloped viral particle, when lytic infection is induced in the infected cell.

The Akata cell used in the present invention is a known human lymphocyte, has about 20 circular EB virus genomes in the nucleus, and is capable of producing a recombinant EB virus generated by a homologous recombination method (Japanese Patent Laid Open No. Hei 8-009971). In addition, since Akata cell can be separated to a clone in which an EB virus genome has been dropped off by passaging the cell for a long period (Japanese Patent Laid Open No. Hei 7-115966). Therefore, a cell clone carrying only a recombinant EB virus genome, wherein a wild type EB virus genome has been dropped off, can be separated by the same method. Moreover, Akata cell can produce a large amount of EB virus by treatment with an anti-human immunoglobulin antibody (Takada K, *Int J Cancer* **33**, 27-32 (1984); Takada K and Ono Y, J *Virol* **63*****,*** 445-449 (1989)).

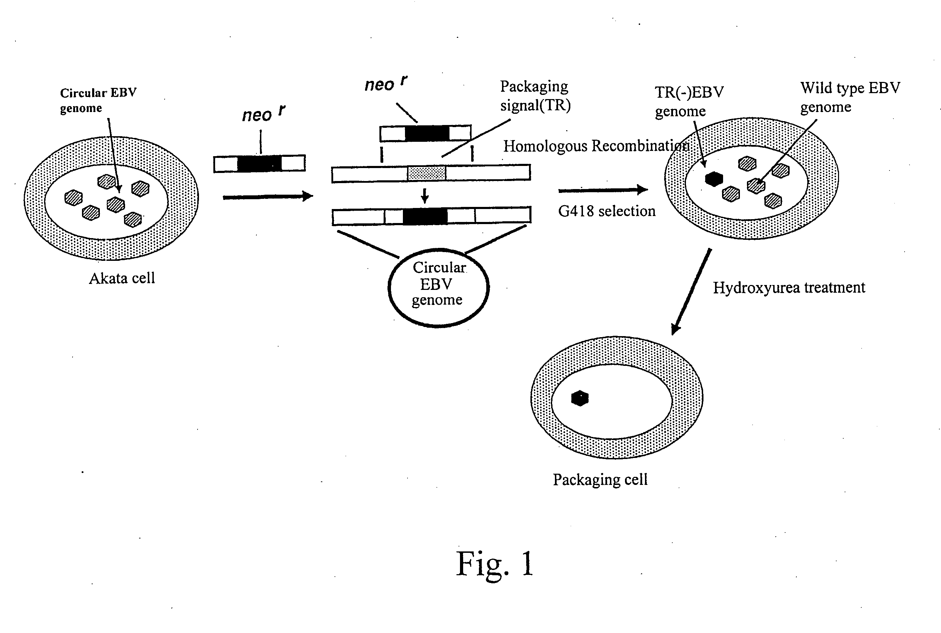

The first embodiment of the method of the present invention is schematically shown in Figure 1. First, a packaging signal of an EB virus is deleted by homologous recombination in an EB virus-positive Akata cell. This step is performed by introducing a gene fragment for homologous recombination, which has regions sandwiching the packaging signal therebetween and lacks the packaging signal itself, into an EB virus-positive Akata cell. The gene fragment for homologous recombination has a length of preferably 30 kb or less, more preferably 20 kb or less. Such a gene fragment can be constructed with the use of the E. *coli* system based on the known EB virus genomic sequence. Introduction of a gene can be carried out by electroporation method, calcium phosphate method, lipofection method, gene gun method and the like. Next, an Akata cell clone having both wild type Akata EB virus genomes and an Akata EB virus genome lacking a packaging signal is separated by utilizing drug resistance. In Figure 1, a neomycin resistant gene is used for selecting the recombinant virus (es). Any selection markers having function in Akata cell may also be utilized. Next, the separated cell clones are treated with hydroxyurea and separated again into cell clones, thereby isolating a packaging cell in which a wild type virus has been dropped off and carrying only a virus lacking the packaging signal. Cloning can also be carried out by limiting dilution after the passage culture without using hydroxyurea.

In the second embodiment of the method of the present invention, the step of deleting a packaging signal of an EB virus by homologous recombination is carried out as follows: by incorporating an EB virus DNA into an *E. coli* artificial chromosome and introduce the DNA into *E. coli,* by preparing an EB virus genome having no packaging signal utilizing gene recombination in *E. coli* cell, and by introducing the resulting EB virus genome into an EB virus-positive Akata cell. It is because an EB virus-positive Akata cell exhibits higher efficiency of foreign gene introduction as compared with an EB virus-negative Akata cell, and can proliferate more stably.

Specifically, first, to prepare a gene fragment for homologous recombination, an *E. coli* artificial chromosome (BAC) vector fragment from pBeloBAC11 (manufactured by Genome Systems Inc.) is inserted into a gene fragment from an EB virus genome. Then, the resulting gene fragment is introduced into an Akata cell having a wild type circular EB virus genome, and thereafter an Akata cell having a homologously recombined EB virus is selected. Next, a plasmid containing both a BAC and an Akata EB virus genome is obtained by transforming E. *coli* with a genomic DNA prepared from the above clone. If an *E*. *coli* gene recombination system is used, deletion and mutation can be introduced at any sites in EB virus genome on this plasmid. Homologous recombination can be taken place in Akata cell by introducing BAC-EB virus DNA thus prepared into Akata cell, wherein the BAC-EB virus DNA lacks a packaging signal.

In the third embodiment of the method of the present invention, the step of deleting a packaging signal of EB virus by homologous recombination is carried out : by incorporating EB viral DNA into an *E*. *coli* artificial chromosome and introduce the DNA into *E*. *coli,* by preparing an EB virus genome lacking a packaging signal utilizing the gene recombination system in *E*. *coli* cell, and by introducing the resulting EB virus genome into an EB virus-negative Akata cell expressing EBNA1. The Akata cell expressing EBNA1 can be cloned from an EB virus-negative Akata cell transfected with a linear DNA in which an EBNA1 gene is ligated with a suitable antibiotic-resistant gene, followed by the selection using the antibiotic. It is thought that efficiency of episome formation is high in an Akata cell expressing EBNA1, since the EBNA1 protein is an essential protein for replication and maintenance of EB virus as an episome in the nucleus.

The present invention also provides a packaging cell produced by the above method. When lytic infection is induced in the Akata packaging cell introduced with an amplicon plasmid, which is prepared by introducing a foreign gene containing a packaging signal (amplicon) into the packaging cell of the present invention, only the amplicon DNA is produced as an EB virus vector coated with a virus envelope by the help of EB virus gene products provided from a TR(-)EB virus genome. That is, the packaging cell of the present invention has a helper function for producing an EB virus vector containing foreign gene(s) and having no viral replication ability.

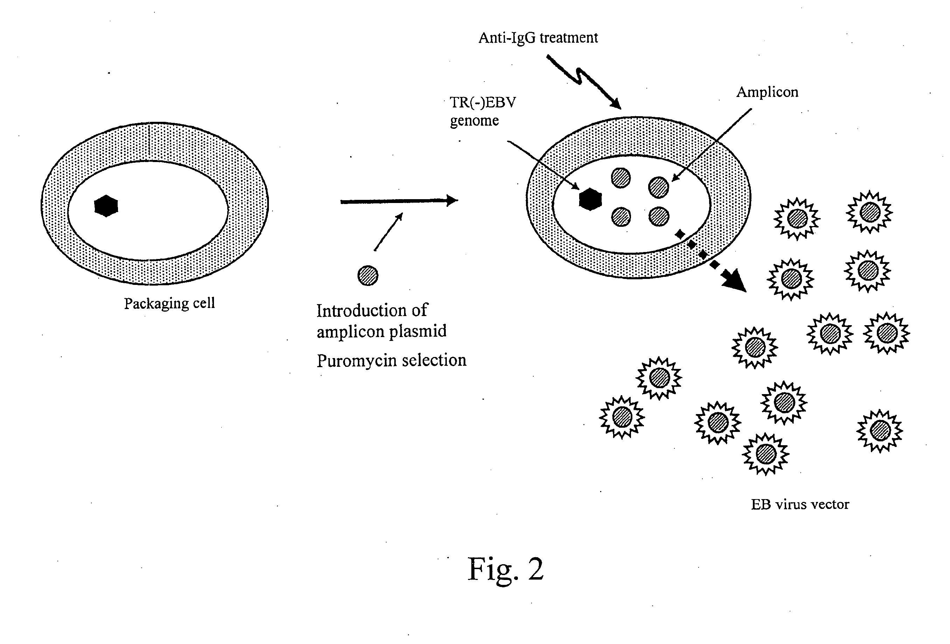

Namely, in another aspect, the present invention provides a method for producing the Akata packaging cell introduced with an amplicon plasmid for producing an EB virus vector without viral replication ability. An outline of this method is shown in Figure 2. An Akata packaging cell containing an amplicon plasmid is cloned by introducing an amplicon plasmid into the packaging cell carrying only an EB virus genome lacking a packaging signal but not carrying a wild type EB virus genome with packaging signal(s), and by selecting the cell with an appropriate drug. Introduction of an amplicon plasmid can be carried out by electroporation, calcium phosphate method, lipofection method, gene gun method and the like.

"Amplicon plasmid" means a plasmid (circular DNA) containing virus-derived genes, however the plasmid lacks the ability to produce a virus. An amplicon plasmid is a circular DNA containing a packaging signal and a virus replication origin, and lacking one or more genes encoding a protein essential for virus replication. Preferably, an amplicon plasmid has selection marker genes. An amplicon plasmid is particularly preferred to have a packaging signal, a replication origin *oriP* during EB virus latent infection, a replication origin *oriLyt* during EB virus lytic infection, and EBNA1. Furthermore, when an EB virus vector is used in order to introduce foreign genes, an amplicon plasmid has the additional foreign genes to be introduced.

In Figure 2, since an amplicon plasmid has a puromycin resistant gene as a selection marker, puromycin was used for selection of the Akata packaging cell introduced with an amplicon. In this case, any selection markers which function in this cell may be utilized. It is preferred to use selection markers different from selection markers for generating the packaging cell.

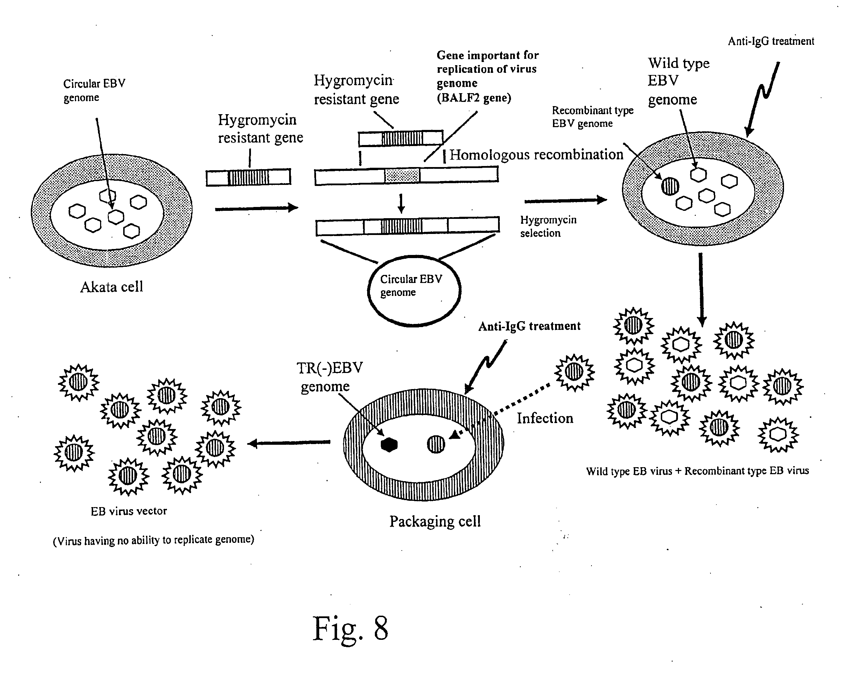

To introduce an amplicon plasmid into a packaging cell, a method based on infection with a virus of which genome is inreplicable may also be used. An outline of this method is shown in Figure 8. A gene important for replication of an EB virus genome is deleted by homologous recombination in an EB virus-positive Akata cell. In Figure 8, a hygromycin resistant gene is used to select a homologous recombinant virus. By treating an Akata cell clone having both wild type virus and isolated homologous recombinant virus was treated with anti-human immunoglobulin, deficient EB virus gene products are supplied from wild type viruses, thereby producing both a homologous recombinant virus and a wild type virus. A packaging cell is infected with the virus liquid solution produced and then cloned with a selection marker (neomycin) used for generating the packaging cell as well as a selection marker (hygromycin) used for generating replication incompetent virus, thereby isolating the Akata packaging cell containing only a TR(-) EB virus gene lacking a packaging signal and an EB virus gene lacking genes important for replication of the viral genome. By treating the resulting Akata packaging cell containing as an amplicon an EB virus gene lacking a gene important for a viral genomic replication, with an anti-human immunoglobulin antibody, only an amplicon plasmid with an EB virus envelope is released as an EB virus vector.

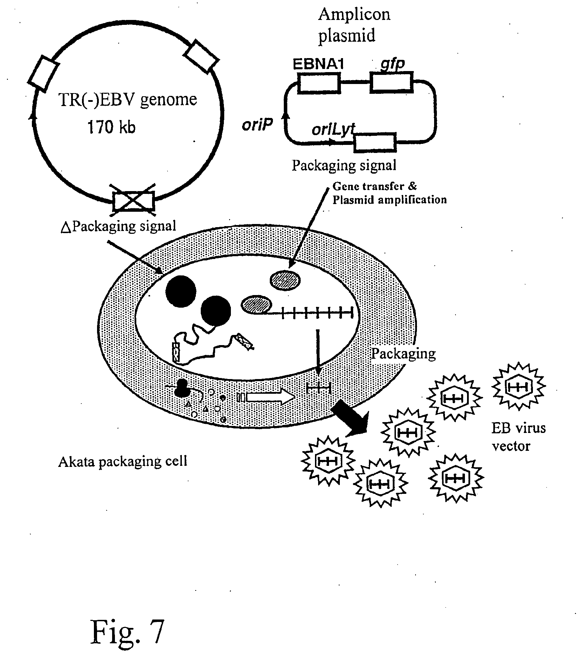

The present invention also provides an Akata packaging cell having an amplicon plasmid produced by the above method. The Akata packaging cell having an amplicon plasmid of the present invention has an ability to produce an amplicon plasmid DNA coated with an EB virus envelope as an EB virus vector by the treatment of the cell with an anti-human immunoglobulin antibody. An EB virus genome is elongated and replicated in a rolling circle-manner. When the packaging signal is deleted, the EB virus genome cannot be excised. Thus, the EB virus genome is not incorporated into a virus particle. On the other hand, a gene of an amplicon plasmid is also elongated in a rolling circle-manner. Thereafter, an amplicon forms a concatemer and is cut at a portion of a packaging signal, followed by packaging at a size of about 170 kb corresponding to the size of an EB virus genome (see, Figure 7). Thus, only an amplicon plasmid covered with an EB virus-envelope is released as an EB virus vector. It is preferable that the Akata packaging cell containing an amplicon plasmid generated by the method of the present invention produces an EB virus vector without viral replication ability at an efficiency of at most 1,000 times or more than that by the conventional method does. And the Akata packaging cell does not produce wild type EB virus.

Namely, in another aspect, the present invention provides a method for producing an EB virus vector without viral replication ability, comprising inducing lytic infection of an Akata packaging cell introduced with an amplicon plasmid, which is produced by the above-mentioned method of the present invention, thereby releasing an EB virus vector covered with a virus envelope. Induction of lytic infection of an Akata packaging cell introduced with an amplicon plasmid can be carried out easily by treatment of the cell with an anti-human immunoglobulin antibody.

The present invention also provides an EB virus vector produced by the above-mentioned method. This EB virus vector contains an amplicon plasmid within an EB virus particle. This EB virus vector is able to infect B-lymphocytes and immortalize them. However, the EB virus vector does not have an ability to replicate itself as a virus particle. Therefore, a human antibody can be produced easily, by infecting a B lymphocyte producing a desired antibody with an EB virus vector of the present invention, by immortalizing the B lymphocyte, and by proliferating the B lymphocyte *in vitro.* Namely, the present invention also provides a method for generating an immortalized B lymphocyte using an EB virus vector of the present invention as well as an immortalized B lymphocyte produced by the method of the present invention. Moreover, by incorporating gene of interest into an amplicon plasmid, it becomes possible to introduce the gene into resting B-lymphocytes in peripheral blood, which has been difficult. By using a promoter capable of expressing a gene only in resting B-lymphocytes, there can be expected B-lymphocyte-specific and high-level expression of the gene.

Entire subject matters of all patent and reference literatures cited explicitly in the present specification are incorporated herein as part of the present specification. Entire subject matters described in the specification and drawings of Japanese Patent Application No. 2002-349467 that serve as the basis for claiming priority of the instant application are also incorporated herein as part of the present specification.

### EXAMPLES

The present invention will be explained further in detail by the following examples, but the present invention is not limited to these examples.

### Example 1

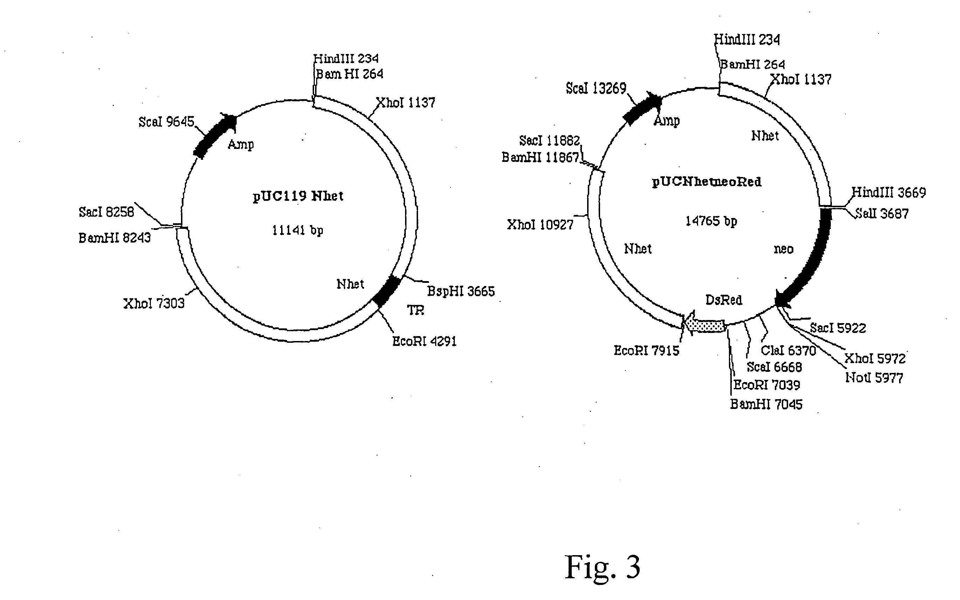

In this example, a packaging signal of Akata EB virus was disrupted based on the method shown in Figure 1. First, an 8 kb Bam-Nhet region of Akata EB virus was cloned into pUC119, to construct pUC119Nhet (Figure 3a). The cloned Bam-Nhet region harboured only one packaging signal (TR). Next, plasmid pUCNhetneoRed for disruption of the gene in which TR had been disrupted was constructed by inserting a neomycin resistant gene *(neo*^{*r*}*)* and a red dye gene (DsRed) to a region between *Bsp*HI and *EcoR*I restriction enzyme site of pUC119Nhet (Figure 3b). This plasmid DNA was introduced into an EB virus-positive Akata cell by the electroporation method.

Subsequently, Akata cell was selected with neomycin and about 500 resistant Akata cell clones were obtained. Then, Akata cell clones having a DNA band of a size which appears in the case of homologous recombination were screened by a DNA hybridization method. A DNA fragment digested with a restriction enzyme *Bam*HI was reacted with a neomycin resistant gene as a probe, to thereby detect a predicted 11.6 kb-band.

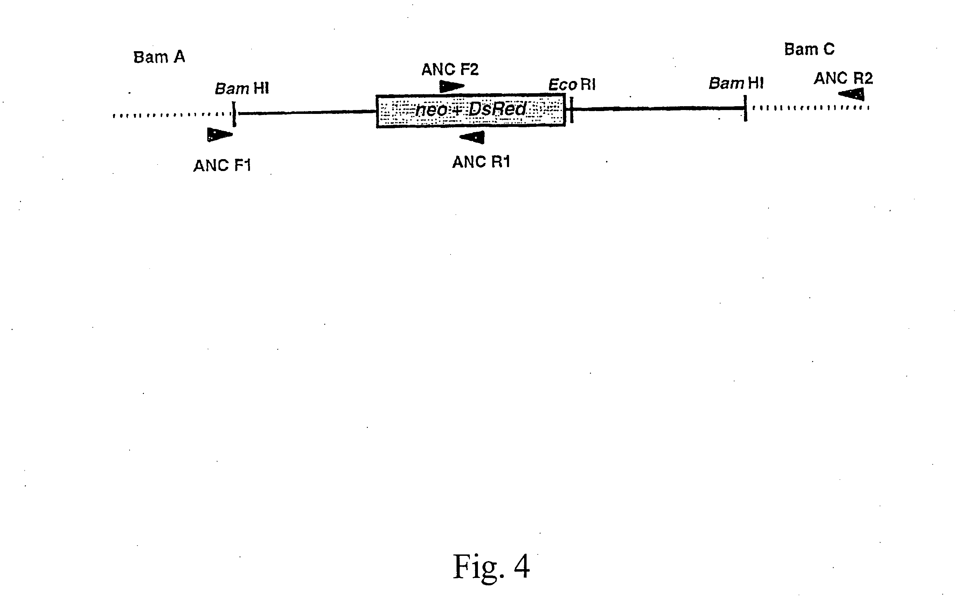

Next, a gene amplification method was performed in order to confirm whether or not homologous recombination was taken place. ANC F1 and ANC R2 are primers designed at the external regions of the 5' end and 3' end of the recombination site, respectively. ANC R1 and ANC F2 are primers designed at the internal region of *neo*^{*r*}*.* Approximate positions and directions of respective primers are shown in Figure 4. In the case where homologous recombination is taken place, a 5.3 kb DNA fragment is amplified by primer pairs of ANC F1 and ANC R1, and a 10.3 kb DNA fragment is amplified by primer pairs of ANC F2 and ANC R2. When homologous recombination is not taken place, no fragment is amplified. With using this method, it was confirmed by this method that 12 clones were carrying a homologous recombinant EB virus.

Next, an Akata cell having only a TR(-) virus genome in which a packaging signal had been disrupted by homologous recombination was isolated. A 119 cell which was a clone carrying a TR(-) EB virus genome was cultured in a selection medium (50% EB virus-negative Akata cell culture supernatant, 0.35 mg/ml G418, 50 µM hydroxyurea) for 10 days. Then, hydroxyurea was removed from the culture and cells were further cultivated for 10 days. The resulting culture was diluted so as to place 0.5 cell per well of a 96-well plate and then cultured. For the proliferated clone, gene amplification was carried out using primer pairs designed at the external regions of TR, to isolate an Akata cell clone in which a band of wild type (2.0 kb) had been dropped off and only a band (5.7 kb) of recombinant type was observed. Further, based on change in a DNA restriction enzyme digestion pattern by a southern blot method, it was confirmed that a packaging cell having only an EB virus lacking a packaging signal was isolated.

### Example 2

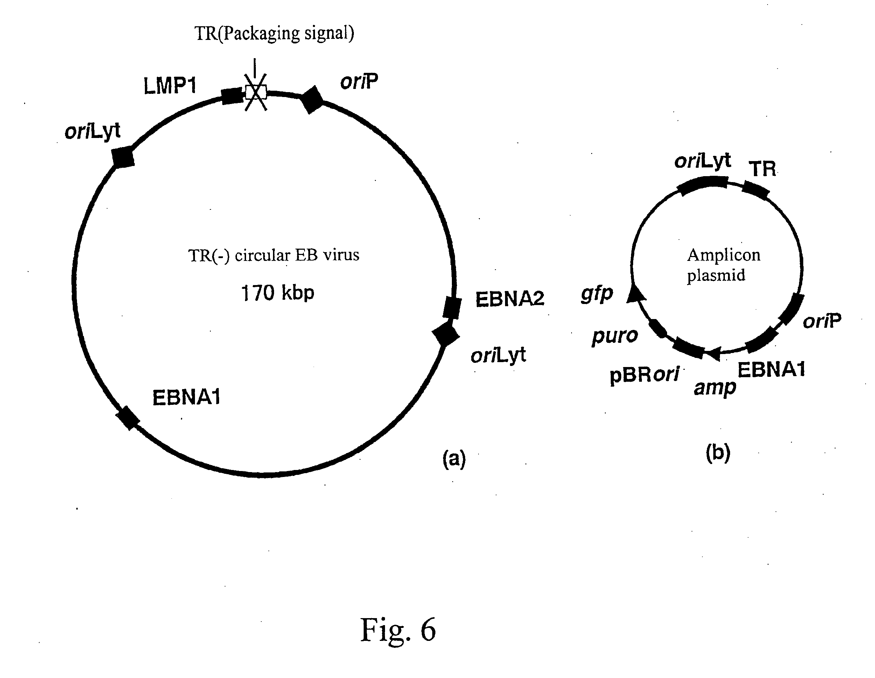

In this example, an amplicon plasmid was prepared. As an amplicon plasmid, it is necessary to have a construction having a packaging signal (TR). Further, it is important for maintenance of the plasmid in the cell to be introduced that the amplicon plasmid should have constructions such as, replication origin *oriP* during an EB virus latent infection period and an EBNA1 gene which trans-activates *oriP.* In addition, in order to produce an EB virus vector from the packaging cell introduced with an amplicon plasmid, by stimulation with an anti-human immunoglobulin antibody, it is necessary for the amplicon plasmid to have a replication origin *oriLyt* during the induced EB virus lytic infection period. Figure 6b shows the construct of the generated amplicon plasmid. The amplicon plasmid (pPsi) is a 21 kb and contains EBNA-1, *oriP,* TR, and *oriLyt,* as EBV genes, wherein a green fluorescent protein (GFP) gene used as a reporter and a puromycin resistant gene used for selection, by which an amplicon plasmid is maintained in a cell, are additionally incorporated. Although a genome of a helper virus is elongated and amplified in a rolling circle-manner, the genome is not excised because of the deficit of TR in its genome. Therefore, the helper virus genome is not incorporated into a virus particle. On the other hand, a gene of an amplicon plasmid is also elongated in a rolling circle-manner. Thereafter, an amplicon forms a concatamer. The concatamer is cleaved at the TR portion at a size of about 170 kb corresponding to the size of an EB virus genome, followed by packaging (Figure 7). That is, an EB virus containing an amplicon plasmid for its gene is released.

### Example 3

In this example, the constructed amplicon plasmid pPsi was transfected into a packaging cell by electroporation. Thereafter, selective culture was carried out in the medium containing 0.35 µg/ml puromycin, 50% EB virus-negative Akata cell culture supernatant, and 0.35 mg/ml G418. The resulting 72 puromycin resistant clones were all GFP-positive. One of the clones was treated with an anti-human immunoglobulin antibody. Thereafter, it was confirmed that a capsid antigen (VCA), which is a constituent protein of an EB virus, was expressed and that a wild type virus was not present in its supernatant. Further, a recombinant EB virus in the supernatant was infected to an EB virus-negative Daudi cell. As the result, it was confirmed that less than 20% of the Daudi cells were infected with a recombinant EB virus and expressed GFP.

### Example 4

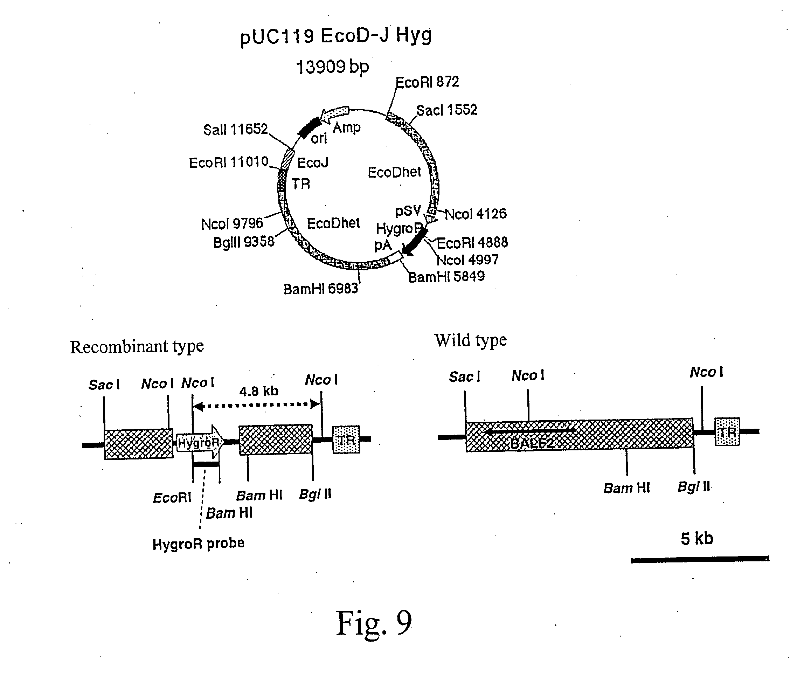

In this example, BALF2 gene encoding EB virus single-stranded DNA binding protein was disrupted by deleting the gene from the Akata EB virus genome through substitution with a hygromycin resistant gene based on the method in Figure 8. First, a plasmid pUC119 EcoD-JHyg for the disruption of the BALF2 gene was prepared by cloning an 11,429 bp *Eco*RI*-Sal*I fragment encompassing the Eco-Dhet and Eco-J regions of Akata EB virus genome into pUC119. From pUC119 EcoD-JHyg, a 2,500 bp *Cla* I*-Eco* RV region was eliminated and substituted with hygromycin resistant gene of 1,853 bp (Figure 9a). A 7,807 bp *Sac* I*-Bgl* II fragment was excised from this plasmid DNA and transfected into an EB virus-positive Akata cell by electroporation.

Subsequently, an Akata cell clone having a DNA band of a size appearing in the case of occurrence of homologous recombination was screened by carrying out hygromycin selection of Akata cell, and then carrying out a DNA hybridization method for about 1,000 resistant clones. A DNA fragment digested with a restriction enzyme *Nco*I was reacted with the hygromycin resistant gene shown in Figure 9b as a probe, to thereby detect a predicted 4.8 kb band on DNA separated from 5 Akata cell clones.

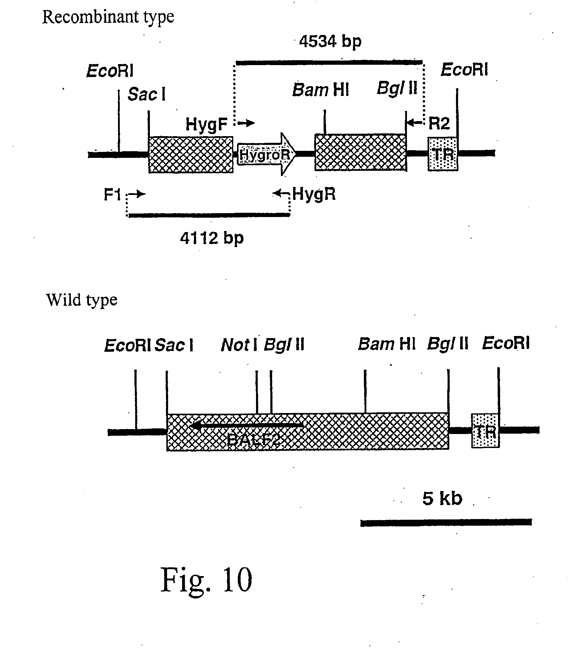

Next, whether or not homologous recombination was taken place was examined by a gene amplification method. F1 and R2 are primers designed at the each external region of 5' end or 3' end of the recombination site, respectively. HygF and HygR are primers designed at the internal region of the hygromycin resistant gene, respectively. Approximate positions and directions of respective primers are shown in Figure 10. In the case where homologous recombination is taken place, a 4,112 bp DNA fragment is amplified by a primer pair of F1 and HygR, and a 4,534 bp DNA fragment is amplified by a primer pair of HygF and R2. In the case where homologous recombination is not taken place, no DNA fragment is amplified. It was confirmed that 2 clones carried a homologous recombinant EB virus by this method.

Next, a clone carrying a homologous recombinant EB virus was treated with an anti-human immunoglobulin antibody, to produce a mixture of virus containing both recombinant type and wild type. The produced virus in the liquid solution was infected to the packaging cell prepared in Example 1, and the cell was subjected to cloning in the presence of neomycin and hygromycin. Subsequently, an Akata packaging cell containing only an EB virus genome lacking BALF2 gene and a TR(-) EB virus genome as a helper, and having no wild type virus genome was separated. This Akata packaging cell was treated with an anti-human immunoglobulin antibody, to recover an EB virus vector containing only a BALF2 gene-deficient EB virus. A half million umbilical cord blood-derived lymphocytes were infected with 10-fold serially diluted BALF2 gene-deficient EB virus liquid solution, each in the volume of 100 µl, and then cultured for 4 weeks. As a result, it was confirmed that an EB virus vector having about 10⁵ TD₅₀/ml transforming activity but having no proliferation ability was produced.

## Claims

1. A method for producing a packaging cell for preparing a system for producing an EB virus vector without viral replication ability, comprising the steps of:
introducing a gene fragment for homologous recombination lacking a packaging signal into Akata cell, thereby deleting packaging signals of EB virus by homologous recombination, and
cloning a packaging cell carrying an EB virus genome lacking a packaging signal, but not carrying a wild type EB virus genome having packaging signals.

2. A method for producing a packaging cell for preparing a system for producing an EB virus vector without viral replication ability, comprising the steps of:
preparing an EB virus genome lacking a packaging signal by the use of *E. coli,*
introducing the EB virus genome into an EB virus-positive Akata cell, and
cloning a packaging cell carrying an EB virus genome lacking a packaging signal, but not carrying a wild type EB virus genome having packaging signals.

3. A method for producing a packaging cell for preparing a system for producing an EB virus vector without viral replication ability, comprising the steps of:
preparing an EB virus genome lacking a packaging signal by the use of *E*. *coli,*
introducing the EB virus genome into an EB virus-negative Akata cell expressing EBNA1, and
cloning a packaging cell carrying an EB virus genome lacking a packaging signal, but not carrying a wild type EB virus genome having packaging signals.

4. A method for producing an Akata packaging cell introduced with an amplicon plasmid, which is used for producing an EB virus vector without viral replication ability, comprising the step of:
introducing an amplicon plasmid having a packaging signal, but lacking viral replication ability into a packaging cell obtained by the method of any one of claims 1 to 3.

5. A method for producing an EB virus vector without viral replication ability, comprising inducing lytic infection of an Akata packaging cell introduced with amplicon plasmid, which is produced by the method of claim 4, thereby allowing an EB virus vector covered with a virus envelope to be released.

6. A method for producing an immortalized B lymphocyte, comprising inducing lytic infection of an Akata packaging cell introduced with amplicon plasmid, which is produced by the method of claim 4, thereby allowing an EB virus vector covered with a virus envelope to be released, and
infecting a B lymphocyte with the resulting EB virus vector.
